# EUROPEAN PATENT APPLICATION

(11) **EP 4 321 596 A1**
(43) Date of publication of application: **14.02.2024**
(21) Application number: 22190306.5
(22) Date of filing: 12.08.2022
(51) Int. Cl.: C10G 2/00, C01B 3/22, C07C 29/151, C07C 1/12

(54) **FISCHER-TROPSCH PRODUCTION OF HYDROCARBONS FROM CARBON DIOXIDE THROUGH METHANOL**

(71) Applicant: BP P.L.C., London SW1Y 4PD (GB)
(72) Inventor: PATERSON, Alexander James, London, SW1Y 4PD (GB); SUNLEY, Glenn, London, SW1Y 4PD (GB)
(74) Representative: Hamer, Christopher K.

(57) **Abstract**

The present disclosure relates generally to processes for performing an integrated Fischer-Tropsch synthesis of hydrocarbons using methanol. In particular, the disclosure relates to a process comprising: providing a first feed stream comprising H₂ and CO₂; contacting the first feed stream with a hydrogenation catalyst for form a first product stream comprising methanol; providing a second feed stream comprising at least a portion of the methanol of the first product stream; contacting the second feed stream with a methanol decomposition catalyst to form a second product stream comprising CO and H₂; providing a third feed stream comprising H₂ and at least a portion of the CO of the second product stream; contacting the third feed stream with a Fischer-Tropsch catalyst to provide a third product stream comprising C₅₊ hydrocarbons.

## Description

### BACKGROUND OF THE DISCLOSURE

### FIELD

The present disclosure relates to integrated processes for the Fischer-Tropsch synthesis of hydrocarbons from carbon dioxide.

### TECHNICAL BACKGROUND

The conversion of synthesis gas (i.e., a mixture of carbon monoxide and hydrogen, also known as syngas) into hydrocarbons by the Fischer-Tropsch process has been known for decades, but has historically lagged in performance compared to other hydrocarbon synthesis techniques. The growing importance of alternative energy sources has resulted in renewed interest in the Fischer-Tropsch (FT) process as it allows a direct and environmentally-acceptable route to high-quality fuels and feedstock chemicals.

FT processes are known for producing linear hydrocarbons for use in fuels, as well as oxygenates that can be useful in fuels and can also serve as valuable feedstock chemicals. The hydrocarbon fuel derived from FT processes is typically better able to meet increasingly stringent environmental regulations compared to conventional refinery-produced fuels, as FT-derived fuels typically have lower contents of sulfur, nitrogen, and aromatic compounds, which contribute to the emission of potent pollutants such as SO₂, NOₓ, and particulates. Products derived from FT processes often have a higher octane rating than hydrocarbons and thus burn more completely, thereby reducing the environmental impact of such a fuel. Alcohols, olefins and other oxygenates obtained may also be used as reagents in other processes, such as in the synthesis of lubricants.

Synthesis gas is conventionally produced from fossil fuel sources, primarily through the gasification of coal. However, syngas suffers from poor handling properties, as it must be transported in the low-density vapor phase, or liquefied under high pressure.

Accordingly, there exists a need to develop improved protocols for the production and handling of syngas and subsequent use of syngas in Fischer-Tropsch synthesis reaction.

### SUMMARY

The inventors have identified processes to efficiently convert CO₂ to hydrocarbons through the intermediacy of methanol (MeOH) and syngas. Advantageously, this process can proceed at lower temperature as compared to conventional synthetic routes, and does not require a reverse water-gas shift (rWGS) process.

Thus, in one aspect, the present disclosure provides a process for performing an integrated Fischer-Tropsch synthesis, the method comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion of the methanol to form a second product stream comprising CO and H₂;
providing a third feed stream comprising H₂ and at least a portion of the CO of the second product stream;
contacting the third feed stream with a Fischer-Tropsch catalyst to perform a Fischer-Tropsch synthesis to provide a third product stream comprising C₅₊ hydrocarbons.

Other aspects of the disclosure will be apparent to those skilled in the art in view of the description that follows.

### BRIEF DESCRIPTION OF THE DRAWINGS

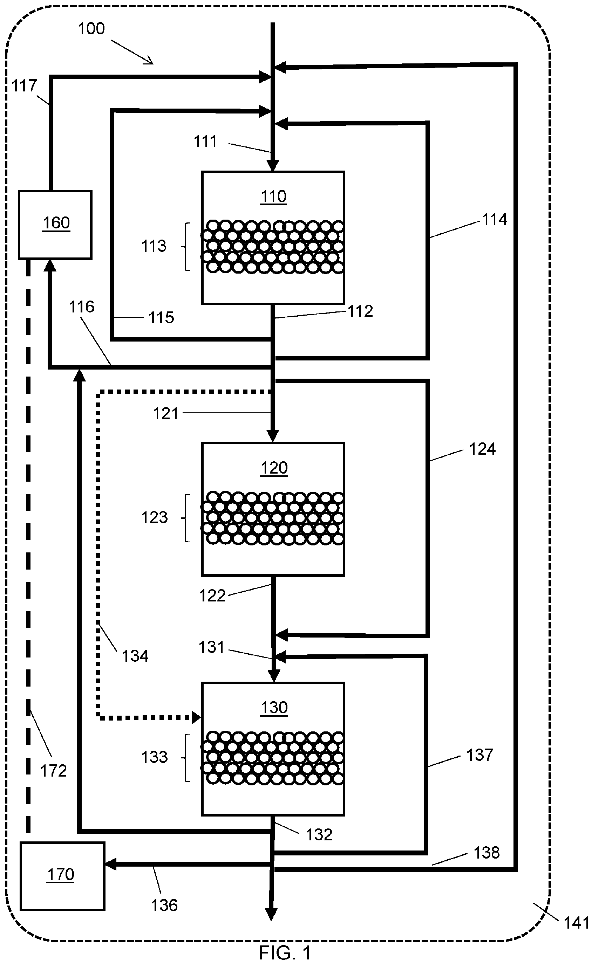

FIGS. 1-5 provide process schematics according to example embodiments of the disclosure.

### DETAILED DESCRIPTION

The present disclosure relates to techniques for preparing hydrocarbons from carbon dioxide. Carbon dioxide is a widely available gas (currently present in the atmosphere at about 400 ppm) that is inert to many transformations. Additionally, the tendency of carbon dioxide to absorb infrared radiation has led to its designation as a greenhouse gas. Thus, there is a need to develop economical processes that utilize carbon dioxide, especially waste carbon dioxide that would otherwise be added to the ever-rising concentration of carbon dioxide in the atmosphere. Carbon dioxide is produced by many industries where it can be captured as a point source to be used as a feedstock, for example from facilities involved in steel production, fossil fuel power generation, cement production, fermentation processes or fertilizer production. Carbon dioxide may also be sourced from direct air capture projects. Advantageously, hydrocarbon synthesis processes that use waste carbon dioxide as a feed have the potential to be low-carbon, carbon neutral, or even have a negative carbon footprint. One way to achieve this is to transform carbon dioxide and hydrogen into methanol. Methanol, being a liquid at ambient temperatures and pressures, may optionally be transported through low-pressure pipelines, ships or trucks, and thus generally allows for increased transportation efficiency as a liquid. Methanol may then be subjected to a methanol decomposition step in order to generate H₂ and CO in a 2:1 ratio. Mixtures of H₂ and CO are commonly referred to as "syngas" and find wide-ranging utility. At least the CO of the methanol decomposition product can then be utilized in a Fischer-Tropsch synthesis reaction in order to generate hydrocarbons; the H₂ from the methanol decomposition is desirably also used in the Fischer-Tropsch synthesis. For example, some Fischer-Tropsch processes operate at a H₂:CO ratio of 2:1 or greater, such at 2.2:1 or 3:1. In such cases, the H₂ and CO mixture from methanol decomposition may be supplemented by a hydrogen source, such as from a recycle stream or green hydrogen produced with renewable energy, to form the Fischer-Tropsch reaction mixture.

Accordingly, in one aspect, the present disclosure provides a process for performing an integrated Fischer-Tropsch synthesis, the method comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion the methanol to form a second product stream comprising CO and H₂;
providing a third feed stream comprising H₂ and at least a portion of the CO of the second product stream;
contacting the third feed stream with a Fischer-Tropsch catalyst to perform a Fischer-Tropsch synthesis to provide a third product stream comprising C₅₊ hydrocarbons.

As used herein, a "feed stream" is used to mean the total material input to a process step, e.g., CO₂ hydrogenation, methanol decomposition, or Fischer-Tropsch reaction, regardless of whether provided in a single physical stream or multiple physical streams, and whether through a single inlet or multiple inlets. For example, H₂ and CO₂ of the first feed stream can be provided to a hydrogenation reactor in a single physical stream (e.g., in a single pipe to the reactor), or in multiple physical streams (e.g., separate inlets for CO₂ and H₂, or one inlet for fresh CO₂ and H₂ and another for recycled CO₂ and/or H₂). Similarly, a "product stream" is used to mean the total material output from a process step, e.g., hydrogenation of carbon dioxide, methanol decomposition, or Fischer-Tropsch reaction, regardless of whether provided in a single physical stream or multiple physical streams, and whether through a single reactor outlet or multiple reactor outlets.

The first feed stream comprises H₂ and CO₂ for subsequent contacting with a hydrogenation catalyst for the production of methanol. Accordingly, in various embodiments as otherwise described herein, the first feed stream includes at least 10 mol% H₂. For example, in various embodiments, the first feed stream includes at least 20 mol% H₂, e.g., at least 30 mol% H₂. In various embodiments as otherwise described herein, the first feed stream includes at least 5 mol% CO₂. For example, in various embodiments, the first feed stream includes at least 10 mol% CO₂, e.g., at least 15 mol% CO₂.

In general, CO₂ hydrogenation to produce methanol proceeds according to the reaction: CO₂ + 3H₂ → CH₃OH + H₂O. Accordingly, in various embodiments as otherwise described herein, the ratio of H₂:CO₂ of the first feed stream is at least 1:1, e.g., at least 1.5:1, on a molar basis. For example, in various embodiments as otherwise described herein, the ratio of H₂:CO₂ of the first feed stream is at least 2:1, e.g., at least 2.5:1, or at least 3:1, or at least 4:1, or at least 5:1, or at least 6:1, or at least 8:1. In particular embodiments, the ratio of H₂:CO₂ of the first feed stream is no more than 25:1, e.g., no more than 20:1, or no more than 15:1, or 12:1.

In some embodiments, the first feed stream comprises other gases in addition to CO₂ and H₂. For example, in various embodiments, the first feed stream further comprises one or more of CO, CH₄, and N₂. For example, in particular embodiments, the first feed stream comprises other gases in addition to CO₂ and H₂ (e.g., one or more of CO, CH₄, and N₂) in an amount up to 70 mol%. In various embodiments, the first feed stream comprises no more than 1% O₂, for example, no more than 0.1% O₂, or no more than 0.01% O₂, or substantially no O₂.

As otherwise described herein, the first feed stream is contacted with a hydrogenation catalyst. The hydrogenation catalyst may be selected by the person of ordinary skill in the art. Examples of suitable catalysts include Cu/ZnO catalysts, for example supported on aluminum oxide or zirconium oxide.

Advantageously, the CO₂ hydrogenation reaction may be performed at a relatively low temperature, leading to increased energy efficiency and integration of the overall process. For example, in various embodiments as otherwise described herein, the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. In various embodiments, the contacting of the first feed stream with the hydrogenation catalyst is performed at a pressure of no more than 100 bar, for example, no more than 80 bar, or no more than 60 bar.

As described herein, the hydrogenation of CO₂ advantageously produces methanol with high selectivity. Accordingly, in various embodiments as otherwise described herein, the hydrogenation of CO₂ is performed with a selectivity of at least 50% for methanol, e.g., at least 55%, or at least 60%. For example, in particular embodiments, the hydrogenation of CO₂ is performed with a selectivity of at least 65% for methanol (e.g., at least 70%, or at least 75%, or at least 80% for methanol). Advantageously, the hydrogenation of CO₂ can be performed with a low selectivity for methane. In various embodiments as otherwise described herein, the hydrogenation of CO₂ is performed with a selectivity of no more than 20% for methane, e.g., no more than 10%, or no more than 5% for methane. As the person of ordinary skill in the art will understand, "selectivity" for a given species is the fraction of the carbon-containing reactant that reacts that is converted to that species, i.e., not counting unreacted material.

Importantly, the CO₂ hydrogenation reaction as described herein is not a reverse water-gas shift reaction. As known in the art, the reverse water-gas shift reaction transforms CO₂ and H₂ into CO and H₂O. Accordingly, in various embodiments as otherwise described herein, the hydrogenation of CO₂ is performed with a selectivity of no more than 20% for CO, e.g., no more than 10%, or no more than 5% for CO. In particular embodiments, the hydrogenation of CO₂ is performed with a selectivity of no more than 2% for CO, or 1% for CO.

During the CO₂ hydrogenation reaction, at least a portion of the CO₂ is hydrogenated into MeOH and H₂O. Advantageously, this process may be performed with a relatively high conversion of CO₂.Accordingly, in various embodiments as otherwise described herein, the hydrogenation of CO₂ is performed with a conversion of CO₂ of at least 25%, e.g. at least 35%, e.g., at least 45% or at least 50%.

The person of ordinary skill in the art will be familiar with catalytic methods for the hydrogenation of carbon dioxide to methanol. Various examples of catalysts and catalytic processes are described in R.Guil-Lopez et al., Materials, 12, 3902 (2019); Xiao et al., In: Aresta et al. (eds) An Economy Based on Carbon Dioxide and Water (2019); Marlin et al., Front. Chem. (2018); Rodriguez et al., ACS Cat. 5(11), 6696 (2015); Choundhury, Chem. Cat. Chem. 4(5), 609 (2012), each of which is hereby incorporated herein by reference in its entirety.

The CO₂ hydrogenation produces a first product stream. In various embodiments as otherwise described herein, the first product stream includes at least 15 mol% methanol, e.g., at least 25 mol%, or at least 35 mol% methanol. As described herein, the CO₂ hydrogenation also has advantageously low selectivity for other products such as methane and/or carbon monoxide. Accordingly, in various embodiments as otherwise described herein, the first product stream includes no more than 10 mol% methane, e.g., no more than 5 mol% methane, or no more than 2 mol% methane. In various embodiments as otherwise described herein, the first product stream includes no more than 10 mol% CO, e.g., no more than 5 mol% CO, or no more than 2 mol% CO. Of course, in some embodiments, there may be more methane and/or CO present, for example, when provided as part of the first feed stream, e.g., as recycle from the Fischer-Tropsch output, As would be understood by the skilled person, the composition of the first product stream as disclosed herein is calculated exclusive of inert gas or unreacted CO₂ and/or H₂.

As described herein, the CO₂ hydrogenation step produces both methanol and water. In some embodiments, the formed water can be deleterious to subsequent processes. For example, excess water present in the methanol decomposition process step can undesirably cause CO to be converted back to CO₂ through the water-gas shift reaction. Additionally, adventitious water may react with methanol in a methanol steam reforming reaction to form CO₂ and hydrogen, undermining process efficiency. Accordingly, in various embodiments as otherwise described herein, the process further comprises separating at least a portion of water from the first product stream. For example, in particular embodiments, the process further comprises separating at least 50%, or at least 75%, or at least 90%, or at least 95%, or at least 99%, or at least 99.5% of the water in the first product stream. In various embodiments as otherwise described herein, the portion of the first product stream that is included in the second feed stream has a water content of no more than 10 mol%, e.g., no more than 2 mol%, or no more than 1 mol%, or no more than 0.5 mol%.

In embodiments in which water is separated from the CO₂ hydrogenation step, the water may be disposed of as waste, or recycled into other processes. For example, in various embodiments, the separated water is directed to an electrolysis reactor for the formation of H₂ gas, e.g., for use in this integrated process or in other processes. In certain embodiments, the separated water is subjected to a purification step before introduction into the electrolysis reactor.

In various embodiments as otherwise described herein, not all H₂ gas input to the CO₂ hydrogenation is reacted. Accordingly, in such embodiments, the process may further comprise separating at least a portion of H₂ from the first product stream. For example, in particular embodiments as otherwise described herein, the process further comprises separating at least 50%, or at least 60%, or at least 75%, or at least 90%, or at least 95% of the H₂ in the first product stream. The separated H₂ may be optionally purified, and then optionally recycled. In various embodiments, the at least a portion of the separated H₂ may be recycled to the first feed stream, and/or may be directed to another reactor. For example, in various embodiments, the at least a portion of the separated H₂ is directed to a Fischer-Tropsch reactor feed stream (e.g., at least a portion of the H₂ separated from the first product stream is provided to the third feed stream). In particular embodiments, the process further comprises activating the Fischer Tropsch catalyst using at least a portion of the H₂ separated from the first product stream. Additionally or alternatively, at least a portion of the H₂ separated from the first product stream may be used to activate a methanol decomposition catalyst.

Advantageously, some processes described herein may be integrated for increased efficiency. For example, in various embodiments as otherwise described herein, the contacting of the second feed stream with the methanol decomposition catalyst and the contacting of the first feed stream with the hydrogenation catalyst are performed in the same plant. As would be appreciated by the person of ordinary skill in the art, the "same plant" signifies that the two processes occur within a small geographical area or property, so that it would be economical to directly connect the two without a significant intervening transportation step. For example, in particular embodiments as otherwise described herein, at least a portion of methanol of the first product stream may be provided directly to the second feed stream.

As described herein, there are a number of uses of hydrogen from the first product stream. Any or all of them may be exploited in a particular real-world process. Notably, the inventors recognize that the presence of hydrogen in the second feed stream may work against the conversion of methanol to carbon monoxide (i.e., because hydrogen is a co-product in that reaction, presence of hydrogen in the second feed stream would force equilibrium in the undesired reverse direction). Accordingly, in various embodiments, the portion of the first product stream that is provided to the second feed stream includes no more than 50% of the hydrogen of the first product stream, e.g., no more than 25%, no more than 10%, or no more than 5% of the hydrogen of the first product stream.

The portion of the first product stream that is provided to the second feed stream may be advantageously be at elevated temperature and/or pressure. This may reduce capital costs by mitigating the heating and/or pressurization needs of the second feed stream. Accordingly, in various embodiments as otherwise described herein, the portion of the first product stream that is provided to the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 C, or 300-400 °C. In particular embodiments, the portion of the first product stream that is provided to the second feed stream is at a pressure of 0 to 50 barg, e.g., 1 to 50 barg.

Compared to gaseous compositions, compositions that are liquid at ambient temperature and pressure generally possess lower capital expenditure for handling due to their increased density and decreased need for high pressures and/or low temperatures. The present inventors have noted that the methanol product of the CO₂ hydrogenation can be simply stored and/or transported. Accordingly, in various embodiments as otherwise described herein, the contacting of the second feed stream with the methanol decomposition catalyst and the contacting of the first feed stream with the hydrogenation catalyst are performed in different plants. As used herein, a different "plant" is not merely a different catalyst bed or different vessel, but rather a different facility than the facility in which the carbon dioxide hydrogenation is performed. In such embodiments, the plant can be, e.g., at least 1 km away from the facility in which the hydrogenation is performed. In various such embodiments, the methanol from the first product stream may be stored and/or transported before provision to the second feed stream.

In various embodiments as otherwise described herein, at least a portion of the methanol of the first product stream is stored (e.g., in one or more tanks) before provision to the second feed stream. The storage may be for any suitable time. In various embodiments, the stored portion of the methanol of the first product stream is stored at least one day, e.g., at least two days, or at least three days, or at least 1 week. In particular embodiments, the stored portion of the methanol is transported at least 1 km during storage, e.g., at least 2 km, or at least 10 km. In some embodiments, the methanol may be transported via pipeline.

The second feed stream introduces methanol to for decomposition into CO and H₂. Methanol decomposition is a distinct process from methanol reforming. In methanol reforming, methanol and water are reacted to produce CO₂ and H₂. Accordingly, in various embodiments as otherwise described herein, methanol reforming is not utilized. For example, in particular embodiments, no more than 10% of the H₂ is derived from methanol reforming (e.g., no more than 5%, or no more than 1%).

The water-gas shift reaction is another method to generate H₂ by reacting carbon monoxide with water. The reaction is reversible as well, where carbon dioxide may be reacted with hydrogen to form carbon monoxide and water. Both of these reactions are conventionally used in the art. Advantageously, the presently disclosed process avoids the use of both the water-gas shift reaction and reverse water-gas shift reaction. Accordingly, in various embodiments as otherwise described herein, less than 10% (e.g., less than 5%, or less than 2%) of each of the H₂, CO, and CO₂ of the process are generated by the water-gas shift reaction or reverse water-gas shift reaction, as appropriate. In various processes as disclosed herein, there is no use of a dedicated water-gas shift reactor or reverse water-gas shift reactor (however, as would be appreciated by the person of ordinary skill in the art, the water-gas shift reaction or reverse water-gas shift reaction can operate to a minor degree as a side reaction during certain processes).

As described herein, methanol decomposition is utilized to form a product stream that includes CO and H₂. Accordingly, in various embodiments as otherwise described herein, the second feed stream comprises at least5 mol% methanol. For example, in particular embodiments, the second feed stream comprises at least 7.5% methanol, e.g., at least10% methanol, or at least 15% methanol, or at least 20% methanol, or at least 25 mol% methanol.

Optionally, the second feed stream may also comprise one or more additional gases, which may be inert or reactive. In such embodiments, the second feed stream may further comprise one or more of H₂, CO, CH₄, CO₂, and N₂. In particular embodiments, the second feed stream comprises an inert carrier gas, wherein the inert carrier gas includes one or more of CH₄, CO₂ and N₂. Additionally or alternatively, H₂ and/or CO may be added to the second feed stream in order to tune the decomposition reaction and/or provide a desired second product stream for use in the Fischer-Tropsch process. In such embodiments, the second feed stream further comprises H₂ and/or CO. In various embodiments as otherwise described herein, one or more of H₂, CO, CH₄, CO₂, and N₂ are be present in second feed stream an amount in the range of up to 50 mol%, e.g., up to 40 mol%, or up to 30 mol%.

In various embodiments, the second feed stream has a low water content. Any water present can react with CO to be at least partially converted to CO₂ and H₂ through the water-gas shift reaction, and water can also react with methanol via reforming to provide H₂ and CO₂.The generated H₂, according to the reaction equilibrium of methanol decomposition, would then disfavor the decomposition of methanol to CO and H₂. Accordingly, in various embodiments as otherwise described herein, the second feed stream has a concentration of water of no more than 5 mol%, e.g., no more than 2 mol%, or no more than 1 mol %, or no more than 0.5 mol%, or no more than 0.3 mol%, or no more than 0.2 mol%, or no more than 0.1 mol%.

The second feed stream may be provided at elevated temperature. For example, in various embodiments as otherwise described herein, the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

The second feed stream is contacted with a methanol decomposition catalyst. Any suitable decomposition catalyst may be used; a variety are known in the art. For example, the decomposition catalyst may include a transition metal, for example, Ni, Co, Rh, Ir, Cu, Pt, Ru, or mixtures thereof. The decomposition catalyst may be a supported catalyst, wherein the support is a refractory oxide, such as oxidized diamond, silica, zirconia, ceria, titania, alumina, or magnesia. In some embodiments, the methanol decomposition catalyst is a copper/zinc oxide catalyst, e.g., on alumina.

The contacting of the second feed stream with the methanol decomposition catalyst occurs at a temperature suitable to effect efficient methanol decomposition. In various embodiments as otherwise described herein, the contacting of the second feed stream with the methanol decomposition catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. The contacting may be performed at a variety of suitable pressures, for example, in the range of 0 to 50 barg. In various embodiments as otherwise described herein, the contacting with the methanol decomposition catalyst may be integrated with a Fischer-Tropsch reactor. Accordingly, in such embodiments, the contacting with the methanol decomposition catalyst may be performed at relatively high pressures, for example, in the range of up to 50 barg, e.g., 20 to 40 barg.

As described herein, the contacting of the second feed stream with the methanol decomposition catalyst may be performed in the same plant as the contacting of the first feed stream with the hydrogenation catalyst. In such examples, it may be advantageous to coordinate the temperatures of the methanol decomposition reaction and the hydrogenation reaction in order to avoid excessive heating and cooling capital costs and energy costs. Accordingly, in particular embodiments as otherwise described herein, the contacting of the second feed stream with the methanol decomposition catalyst is performed at a temperature within 75 °C of a temperature of the contacting of the first feed stream with the hydrogenation catalyst, e.g., within 50 °C.

The methanol decomposition reaction generates CO and H₂, ideally with minimal other products. Accordingly, in various embodiments as otherwise described herein, the decomposition of methanol is performed with a carbon product selectivity of at least 50% for CO, e.g., at least 60%, or at least 70%, or at least 80%, or at least 90% for CO. Advantageously, in various embodiments, the decomposition of methanol is performed with a selectivity of no more than 20% for CO₂, e.g., no more than 15%, or no more than 10%, or no more than 5%. In various embodiments as otherwise described herein, the decomposition of methanol is performed with a conversion of methanol of at least 30%, e.g., at least 40%, or at least 50%, or at least 60%, or at least 65%, or at least 70%, or at least 75%.

As described herein, the decomposition of methanol generates CO and H₂. Accordingly, in various embodiments as otherwise described herein, the second product stream comprises at least 20 mol% total of CO and H₂, e.g., at least 35 mol%, or at least 50 mol%, or at least 65 mol%. Without wishing to be bound by theory, methanol decomposition under these conditions is expected to generated two moles of H₂ per mole of CO. Accordingly, in various embodiments as otherwise described herein, the second product stream has a molar ratio of hydrogen to carbon monoxide in the range of 0.5:1 to 5:1, e.g., 1:1 to 3:1, or 1.5:1 to 2.5:1, or 1.5:1 to 3.5:1. Of course, in other embodiments, this molar ratio may be different, e.g., as a consequence of inclusion of H₂ or CO in the second feed stream.

Advantageously, a proportion of methanol is consumed during the methanol decomposition reaction. Accordingly, in various embodiments as otherwise described herein, the second product stream includes no more than 75 mol% methanol, e.g., no more than 60 mol% methanol, no more than 50 mol% methanol, or no more than 25 mol% methanol.

In some embodiments, further removal of methanol from the second product stream is desired. For example, in various embodiments as otherwise described herein, the process further comprises separating at least a portion of methanol from the second product stream, e.g., separating at least 50%, or at least 75%, or at least 90%, or at least 95% of the methanol from the second product stream. Advantageously, methanol of the second product stream, e.g., at least a portion of the methanol separated from the second product stream, may be transferred or recycled to other processes. For example, in various embodiments as otherwise described herein, the process further comprises recycling to the second feed stream at least a portion of the methanol separated from the second product stream.

The person of ordinary skill in the art will be familiar with catalytic methods for the decomposition of methanol to CO and H₂. Various examples of catalysts and catalytic processes are described in U.S. Patent no. 6,541,142, U.S. Patent no, 9,883,773, and U.S. Patent no. 4,716,859 each of which is hereby incorporated herein by reference in its entirety.

As described herein, the methanol decomposition reaction produces CO and H₂ in a theoretical 2:1 molar ratio, although the actual reaction output may vary. This can result in excess hydrogen for subsequent processes, depending on the desired ratio. And, notably, hydrogen may be present in the second feed stream, further increasing the ratio in the second product stream. Accordingly, in various embodiments as otherwise described herein, the process further comprises separating at least a portion of H₂ from the second product stream. The separated H₂ may be suitable for a variety of processes. For example, in particular embodiments, at least a portion of the separated H₂ from the second product stream is provided to the first feed stream. Additionally or alternatively, in various embodiments as otherwise described herein, the process further comprises activating the methanol decomposition catalyst or the Fischer-Tropsch catalyst using at least a portion of the H₂ separated from the second product stream.

As described herein, the third feed stream comprises at least a portion of the CO of the second product stream. Accordingly, in various embodiments as otherwise described herein, at least 50% of the CO of the second product stream is provided to the third feed stream. For example, in particular embodiments, at least 75% of the CO of the second product stream is provided to the third feed stream, e.g., at least 90%, or at least 95%, or at least 99%. In various embodiments, substantially all of the CO of the second product stream is provided to the third feed stream. Additionally or alternatively, in various embodiments as otherwise described herein, the CO of the third feed stream may be supplied in part from a CO source other than the second product stream.

As noted above, the third feed stream comprises H₂ and CO, and the second product stream includes H₂. In various embodiments as otherwise described herein, at least a portion of the H₂ of the second product stream is provided to the third feed stream. For example, in particular embodiments, at least 50% of H₂ of the second product stream is provided to the third feed stream, e.g., at least 75%, or at least 90%, or at least 95%, or at least 99%. In various embodiments, substantially all of the H₂ of the second product stream is provided to the third feed stream. Additionally or alternatively, in various embodiments as otherwise described herein, the H₂ of the third feed stream may be supplied at least in part from a hydrogen source other than the second product stream.

In various embodiments as otherwise described herein, the third feed stream may include other gases, for example, CO₂, CH₄, or N₂. Other gases, in embodiments where they are included, may be contained in the second product stream and carried through to the third feed stream. But in other embodiments, one or more other gases (e.g., one or more of CO₂, CH₄, N₂) is provided to the third feed stream from a source thereof other than the second product stream.

Advantageously, the portion of the second product stream that is included in the third feed stream may be tuned as desired for the processes of the third feed stream. For example, in various embodiments as otherwise described herein, the portion of the second product stream that is included in the third feed stream has a H₂:CO ratio in the range of 0.5:1 to 5:1, e.g., in the range of 1:1 to 2.5:1.

As otherwise described herein, the third feed steam is contacted with a Fischer-Tropsch catalyst. Accordingly, in various embodiments as otherwise described herein, the third feed steam has a molar H₂:CO appropriate for Fischer-Tropsch synthesis. For example, in various embodiments, the third feed stream has a molar H₂:CO ratio in the range of 0.5:1 to 5:1, e.g., in the range of 1:1 to 2.5:1. In particular embodiments, the third feed stream has a molar H₂:CO ratio of at least 1.2:1. For example, in various embodiments the third feed stream may have a molar H₂:CO ratio in the range of 1.2:1 to 2.5:1, or in the range of 1.4:1 to 2.5:1, or in the range of 1.6:1 to 2.2:1.

The Fischer-Tropsch catalyst may be selected by the person of ordinary skill in the art. In various embodiments as otherwise described herein, the Fischer-Tropsch catalyst comprises cobalt, iron, rhodium, ruthenium, or a combination thereof (e.g., comprises cobalt or iron). In particular embodiments, the Fischer-Tropsch catalyst comprises cobalt in an amount in the range of 2 to 30 wt%, e.g., in the range of 5 to 25 wt%, or in the range of 8 to 20 wt%, calculated as Co(0). In particular embodiments, the Fischer-Tropsch catalyst comprises iron in an amount in the range of 15 to 95 wt%, e.g., 25 to 95 wt%, or 30 to 90 wt%, calculated as Fe(0). In various embodiments as otherwise described herein, the Fischer-Tropsch catalyst further comprises manganese (e.g., comprises manganese in the amount of 0.1 to 15 wt%, or 1 to 10 wt%).

In various embodiments as otherwise described herein, the Fischer-Tropsch catalyst is a supported catalyst, wherein the support comprises at least one of titanium oxide, zirconium oxide, cerium oxide, aluminum oxide, silicon oxide, magnesium oxide, and zinc oxide (e.g., comprises at least one of titanium oxide, aluminum oxide, and silicon oxide). For example, in particular embodiments, the support is a titanium dioxide support, or is an alumina support, or is a silica support. In various embodiments, the support is a shaped particle, e.g., an extrudate. The catalyst can be prepared using methods conventional in the art.

Prior to use, certain catalysts require activation. In various embodiments as otherwise described herein, the Fischer-Tropsch catalyst is activated in a reductive atmosphere. In various embodiments, the catalyst is substantially reduced to generate a reduced catalyst material that is used in the catalysis of the Fischer-Tropsch reaction. In embodiments wherein the Fischer-Tropsch catalyst comprises cobalt, this process results in at least portion of the cobalt being transformed into cobalt metal. Desirably, the reduction results in at least 50% of the cobalt being provided as cobalt(0). The person of ordinary skill in the art can use conventional methods to reduce cobalt catalyst materials (e.g., cobalt oxide- or cobalt hydroxide-based materials) to metallic form. In various embodiments as otherwise described herein, the first reducing agent is hydrogen gas, H₂. The hydrogen gas may be mixed with other gases, such as an inert carrier gas. Examples of such inert carrier gasses include nitrogen, carbon dioxide, argon, or helium. The hydrogen gas may also be mixed with carbon monoxide, with or without one or more additional carrier gasses. In various embodiments, the reduction is effected by contacting the first catalyst material with a first reducing gas, wherein the first reducing gas comprises the first reducing agent, wherein the first reducing gas comprises at least 50 vol% H₂ (e.g., at least 60 vol%, or at least 70 vol%, or at least 80 vol%, or at least 90 vol%, or at least 95 vol%, or essentially 100 vol% H₂). As noted above, H₂ for reduction can come from the first product stream or the second product stream. H₂ can also be separated and recycled from the third product stream for use in reducing the Fischer-Tropsch catalyst.

The temperature of the Fischer-Tropsch synthesis can suitably be in the range of 200-400 °C, such as 200-300 °C, or 210-400 °C, or 210-300 °C, or 220-400 °C, or 220-300 °C. In various embodiments as otherwise described herein, the temperature of the Fischer-Tropsch synthesis is in the range of 200-250 °C, e.g., or 200-240 °C, or 200-230 °C, or 200-220 °C, or 210-250 °C, or 210-240 °C, or 210-230 °C, or 220-250 °C, or 220-240 °C, or 230-250 °C. The pressure of the reaction may suitably be in the range from 10-50 barg e.g., 20-50 barg, or 25-50 barg, or 10-40 barg, or 20-40 barg, or 25-40 barg or 10-35 barg, or 20-35 barg, or 25-35 barg. In particular embodiments, the contacting of the third feed stream with the Fischer-Tropsch catalyst is performed at a pressure in the range of 20-40 barg.

Subject to the limitations described herein, the person of ordinary skill in the art can adapt conventional Fischer-Tropsch catalysts and processes to arrive at the processes of the disclosure. Suitable techniques for catalyst preparation and hydrocarbon synthesis, especially with regard to the synthesis of C₅₊ hydrocarbons and/or oxygenates, are well-known in the art, for example, described in International Patent Application Publication No. 2019/154885, which is hereby incorporated by reference herein in its entirety.

Advantageously, in various embodiments as otherwise described herein, the contacting of the third feed stream with the Fischer-Tropsch catalyst and the contacting of the second feed stream with the methanol decomposition catalyst may be performed in the same plant. This can be, e.g., within different beds in the same reactor, or in different reactors. In such embodiments, the two processes may be integrated together to provide efficient transformation of methanol to hydrocarbons.

The Fischer-Tropsch process is typically used to make C₅₊ hydrocarbons, for example, unsubstituted C₅₊ hydrocarbons (e.g., alkanes and alkenes) and oxygenated C5+ hydrocarbons (e.g., C₅₊ alcohols, aldehydes, ketones, carboxylic acids). The Fischer-Tropsch reaction as described herein is desirably operated with a high selectivity for hydrocarbons. For example, in various embodiments as otherwise described herein, the contacting of the Fischer-Tropsch catalyst with the third feed stream is performed with a C₅₊ selectivity of at least 30%, e.g., at least 50%, or at least 70%. The hydrocarbon composition of the third product stream can vary depending on identify of catalyst and process conditions as known in the art. In various embodiments, the hydrocarbon composition comprises hydrocarbons (e.g., linear hydrocarbons, branched hydrocarbons, saturated or unsaturated hydrocarbons) and oxygenated derivatives thereof. In various embodiments as otherwise described herein, the hydrocarbon composition comprises at least one of alkanes, alkenes, and alcohols.

In general, longer-chain hydrocarbons are more desirable compared to shorter-chain hydrocarbons. Accordingly, in various embodiments as otherwise described herein, the third product stream comprises C₁-C₄ hydrocarbons, and the process further comprises separating at least a portion of the C₁-C₄ hydrocarbons from the third product stream to provide a light hydrocarbon stream. In particular embodiments, the process further comprises oxidizing at least a portion of the light hydrocarbon stream in a partial oxidation reactor to provide a pOX stream comprising CO and/or CO₂, and including at least a portion of the pOX stream in the first feed stream and/or the third feed stream. For example, catalytic partial oxidation of hydrocarbon feedstocks is described in European Patent Application Publication no. 0303438 A1, which is hereby incorporated herein by reference in its entirety. Additionally or alternatively, in particular embodiments, at least a portion of the light hydrocarbon stream may be directed to a steam reforming unit and/or an autothermal reforming unit, such as an electrically heated steam reforming unit. The resulting hydrogen and/or carbon oxides may then be included, at least in part, in the first feed steam and/or the third feed stream as otherwise described herein.

The light hydrocarbon stream comprises numerous components, including short-chain (e.g., C₁-C₄) saturated hydrocarbons, short-chain olefins, and short-chain oxygenates. In particular, olefins (e.g., ethene, propene, butene) and alcohols, may also be recycled to the Fischer-Tropsch reactor in order to generate additional C₅₊ hydrocarbons. Accordingly, in various embodiments as otherwise described herein, the process further comprises including at least a portion of the light hydrocarbon stream in the third feed stream. Additionally or alternatively, at least a portion of the light hydrocarbon stream can be included in the first feed stream and/or the second feed stream, to be passed through to the third feed stream.

In various embodiments, the third product stream may comprise unreacted H₂. In various embodiments as otherwise described herein, the process further comprises separating at least a portion of H₂ of the third product stream. The separated H₂ may then be directed to a process in need thereof. For example, in particular embodiments, at least a portion of the H₂ separated from the third product stream is provided to the first feed stream and/or to the third feed stream. And as mentioned above, at least a portion of H₂ separated from the third product stream can be used to activate the Fischer-Tropsch catalyst.

In various embodiments, the third product stream may comprise unreacted CO. In various embodiments as otherwise described herein, the process further comprises separating at least a portion of CO of the third product stream. The separated CO may then be directed to a process in need thereof. For example, in particular embodiments, at least a portion of the CO separated from the third product stream is provided to the third feed stream.

In various embodiments, the third product stream may comprise CO₂. Accordingly, in various embodiments as otherwise described herein, the process further comprises separating at least a portion of CO₂ from the third product stream. The separated CO₂ may then be directed to a process in need thereof. For example, in particular embodiments, at least a portion of the CO₂ separated from the third product stream is provided to the first feed stream.

It can be desirable to provide from the third product stream a C₅₊ hydrocarbon product stream, i.e., that contains at least 70 wt% C₅₊ hydrocarbons, e.g., at least 80 wt% C₅₊, e.g., at least 85 wt% C₅₊ hydrocarbons. As the person of ordinary skill in the art will appreciate, this can be performed by separating out at least a portion of other components, such as C₁-C₄ hydrocarbons, CO, CO₂ and H₂, e.g., as described above.

Moreover, it can be desirable in some cases to perform further transformations on the C₅₊ hydrocarbon product stream. Hydroprocessing, i.e., contacting the C₅₊ hydrocarbon product stream with hydrogen and a hydroprocessing catalyst, can be used for this purpose. Accordingly, in various embodiments, a process as otherwise described herein includes hydroprocessing the C₅₊ hydrocarbon product stream by contacting the C₅₊ hydrocarbon product stream with hydrogen and a hydroprocessing catalyst. A variety of hydroprocessing process steps may be used. For example hydrocracking can be used to provide products with lower molecular weight, e.g., to convert higher molecular weight waxes to lower molecular weight products for use as fuels such as aviation fuel, diesel fuel or gasoline. Hydrodeoxygenation can be used to convert oxygenates to non-oxygenated hydrocarbons. Hydroprocessing can also be used to convert olefins to alkanes. Hydroisomerization may be used to alter the mix of hydrocarbon isomers. At least a portion of the H₂ used in the hydroprocessing can be H₂ separated from one or more of the first, second and third product streams as described above.

As the person of ordinary skill in the art will appreciate, the Fischer-Tropsch processes described herein can be used to provide a variety of end products. For example, in various embodiments as otherwise described herein, one or more products are provided from at least a portion of C₅₊ hydrocarbons of the third product stream. The one or more products can be, for example, one or more of a fuel (e.g., a diesel fuel, a gasoline, or an aviation fuel), a wax or an oil (e.g., for use in lubrication or metalworking).

The processes described herein can be performed with high throughputs. For example, in various embodiments as otherwise described herein, the CO₂ hydrogenation, MeOH decomposition, and/or Fischer-Tropsch synthesis is conducted at a GHSV in the range of 2000 hr⁻¹ to 20,000 hr⁻¹. For example, in various such embodiments the contacting is conducted at a GHSV in the range of 4000 hr⁻¹ to 18,000 hr⁻¹, or 6000 hr⁻¹ to 16,000 hr⁻¹, or 8000 hr⁻¹ to 12,000 hr⁻¹, or 2000 hr⁻¹ to 12,000 hr⁻¹, or 4000 hr⁻¹ to 10,000 hr⁻¹, or 6000 hr⁻¹ to 10,000 hr⁻¹, or 2000 hr⁻¹ to 8,000 hr⁻¹, or 4000 hr⁻¹ to 8,000 hr⁻¹, or 2000 hr⁻¹ to 6000 hr⁻¹. Of course, depending on the system, higher and lower space velocities may also be used.

The present inventors have noted that carbon dioxide is a chief input of carbon to the processes described herein, and that carbon dioxide can advantageously be provided by a variety of sources. Importantly, at least part of the carbon dioxide of the first feed stream (e.g., at least 50%, at least 75%, at least 90% or at least 95%) can come from a renewable source.

Carbon dioxide is a common waste material, and often desirable to be removed from waste streams rather than be vented to the atmosphere. Such capture of carbon dioxide is critical to the implementation of many renewable initiatives as it serves to lower the carbon footprint of the associated process. Advantageously, the carbon dioxide utilized in the processes described herein may be carbon dioxide collected from the atmosphere or that would otherwise have been released into the atmosphere, e.g., from a combustion or other industrial process. The carbon dioxide may be captured, where it is collected or absorbed after release from an industrial process, or harvested directly from the atmosphere. Methods of carbon dioxide capture are known to those of skill in the art. In various embodiments, at least a portion of the CO₂ of the first feed stream is from direct air capture. Additionally or alternatively, carbon dioxide is often scrubbed from industrial effluent, especially processes that generate large amounts of carbon dioxide as a byproduct. Accordingly, in various embodiments as otherwise described herein, at least part of the CO₂ of the first feed stream (e.g., at least 50%, at least 75%, at least 90% or at least 95%) is captured from a manufacturing plant, e.g., a bioethanol plant, a steel plant or a cement plant.

As noted above, renewable sources can be used to provide CO₂ to the claimed processes. For example, biomass is an attractive source of renewable carbon dioxide for use in the processes described herein. One source of biomass is agricultural products in the form of dedicated energy crops such as switchgrass, miscanthus, bamboo, sorghum, tall fescue, kochia, wheatgrass, poplar, willow, silver maple, eastern cottonwood, green ash, black walnut, sweetgum, and sycamore. Another biomass source is agricultural waste or agricultural crop residue. Conventional agricultural activities, including the production of food, feed, fiber, and forest products, generate large amounts of waste plant material. Examples of such materials include corn stover, wheat straw, oat straw, barley straw, sorghum stubble, and rice straw. A third biomass source is through forestry residues left after timber operations. Biomass may also be in the form of commercial waste, industrial waste, sewage sludge, and municipal waste, which includes commercial and residential garbage, including yard trimmings, paper and paperboard, plastics, rubber, leather, textiles, and food waste. Accordingly, in various embodiments as otherwise described herein, the at least a portion of the CO₂ of the first feed stream is derived from a renewable source. For example, in particular embodiments, at least a portion (e.g., at least 50%, at least 75%, at least 90% or at least 95%) of the CO₂ of the first feed stream is derived from biomass, for example, agricultural biomass or municipal waste biomass. Additional sources of agricultural biomass will be apparent to one of skill in the art as dictated by local availability, economics, and process compatibility.

To generate carbon dioxide from a carbon-containing material, such as biomass, the material is typically subjected to gasification. Gasification involves heating the material under controlled conditions to generate gaseous streams of carbon monoxide, hydrogen, and carbon dioxide. Controlled amounts of other reactants, such as oxygen and/or steam, may be used to tune the process. Gasification conditions are tuned in accordance with the carbon-containing material being gasified in order to efficiently produce gaseous products. In various embodiments, the carbon dioxide of the first feed stream includes carbon dioxide from gasification of biomass, e.g., at least 50%, at least 75%, at least 90% or at least 95% of the carbon dioxide is from gasification of biomass. The biomass may be any source as described above, or from multiple sources may be combined.

Conventional H₂ gas is most commonly derived from natural gas, often through steam reforming of methane, hydrocarbon partial oxidation, and/or coal gasification. However, each of these methods, as conventionally performed, release significant amounts of CO₂ into the atmosphere and rely on fossil fuels as a starting material and/or energy source. In contrast, other sources of H₂ are known which are preferable from an environmental standpoint. Accordingly, in various embodiments as otherwise described herein, at least a portion of the H₂ of the first feed stream, second feed stream and/or the third feed stream (e.g., at least 50%, at least 75%, at least 90% or at least 95%) is from a renewable resource.

As known in the art, hydrogen can be provided with color-based names according to its source of production. Examples of types of hydrogen include green hydrogen, blue hydrogen, grey hydrogen, black hydrogen, brown hydrogen, pink hydrogen, turquoise hydrogen, yellow hydrogen, and/or white hydrogen.

One potential source of green hydrogen is through the electrolysis of water. Numerous methods of electrolysis are known in the art. For example, electrolysis may be performed on pure water to produce hydrogen gas and oxygen gas, or on other solutions, such as saline solution, to produce hydrogen gas and another product (e.g., chlorine gas). In particular embodiments, the hydrogen is formed through the electrolysis of a saline solution. Water electrolysis is described further in U.S. Patent No. 4,312,720, U.S. Patent No. 4,021,323, and U.S. Patent No. 4,094,751, each of which is incorporated by reference in their entirety.

To qualify as green hydrogen, the electrical power used for the water electrolysis must be from a renewable source, that is, a source that does not depend on fossil fuel combustion. Example sources of renewable power include solar power through photovoltaic capture or solar thermal technology, wind power, geothermal energy capture, hydroelectric energy, or other renewable sources. Hydrogen that uses solar power for water electrolysis is sometimes called yellow hydrogen. Appropriate renewable energy sources are known to those of skill in the art, and may optionally be selected through certification by an appropriate agency. Accordingly, in various embodiments as otherwise described herein, at least a portion of the H₂ of the first feed stream and/or the third feed stream is green hydrogen and/or yellow hydrogen. For example, in particular embodiments, the process further comprises providing at least a portion of the H₂ of the first feed stream and/or the third feed stream by electrolysis of water. In various embodiments, the electrolysis of water is performed using electricity at least partially derived from a renewable source.

Certain recycle streams may also be adapted to provide power for water electrolysis as appropriate. For example, in various embodiments as otherwise described herein, the electrolysis of water is performed using electricity at least partially generated from steam made by combustion of a light hydrocarbon stream provided at least partially from the third product stream.

Water electrolysis conventionally produces both H₂ and O₂ gas. As otherwise described herein, in some embodiments, the process further comprises oxidizing at least a portion of the light hydrocarbon stream (e.g., derived from the third product stream) to provide a pOX stream. In such embodiments, the oxidation may be performed using O₂. Accordingly, in particular embodiments, at least a portion of the O₂ generated by water electrolysis is provided to the partial oxidation reactor.

Blue hydrogen is defined as hydrogen gas produced with some reliance on fossil fuels, but in a process that is overall carbon neutral (i.e., does not result in any net introduction of carbon dioxide into the atmosphere). In various embodiments as otherwise described herein, the hydrogen utilized in the processes as otherwise described herein comprises blue hydrogen. An example of blue hydrogen is hydrogen gas produced from fossil fuel-derived hydrocarbons such as methane gas, wherein the resulting carbon product is captured or otherwise utilized. For example, steam reforming of methane may be conducted to produce three moles of hydrogen gas and one mole of carbon monoxide for each mole of methane. Methane steam reforming is highly endothermic, requiring significant energy input. Of course, the energy required to perform these processes must be sources from renewable sources, or sources with adequate carbon capture technology. Accordingly, in various embodiments as otherwise described herein, at least a portion of the hydrogen (e.g., at least 50%, at least 75%, at least 90% or at least 95%) is formed through the steam reforming of methane. Steam reforming of methane to produce hydrogen is discussed in International Patent Application Publication no. 2004/022480, which is herein incorporated by reference in its entirety. Accordingly, in various embodiments as otherwise described herein, at least a portion of the H₂ of the first feed stream and/or the third feed stream is blue hydrogen.

Grey hydrogen is the source of most conventional H₂. Grey hydrogen is created from natural gas/methane through steam reforming, but, in contrast to blue hydrogen, none of the resulting greenhouse gases (e.g., CO₂) is captured. In various embodiments as otherwise described herein, at least a portion of the H₂ of the first feed stream and/or the third feed stream is grey hydrogen.

Black hydrogen and brown hydrogen are produced from black coal or brown coal, respectively, often through gasification thereof without any carbon capture. In various embodiments as otherwise described herein, at least a portion of the H₂ of the first feed stream and/or the third feed stream is black hydrogen and/or brown hydrogen.

Pink hydrogen is generated through water electrolysis where the required energy for the electrolysis is generated by nuclear power. In various embodiments as otherwise described herein, at least a portion of the H₂ of the first feed stream and/or the third feed stream is pink hydrogen. Pink hydrogen is also known as purple hydrogen or red hydrogen.

Turquoise hydrogen is generated by methane pyrolysis, where the byproducts are hydrogen gas and solid elemental carbon. In various embodiments as otherwise described herein, at least a portion of the H₂ of the first feed stream and/or the third feed stream is turquoise hydrogen.

White hydrogen is naturally-occurring hydrogen which can be released through tracking. In various embodiments as otherwise described herein, at least a portion of the H₂ of the first feed stream and/or the third feed stream is white hydrogen.

As described herein, the disclosed process, in various embodiments, includes three central reactions: the hydrogenation of CO₂, methanol decomposition, and Fischer-Tropsch synthesis. As described herein, these reactions may be advantageously carried out in different spatial arrangements relative to each other. As known in the art, reaction zones are comprised of one or more catalyst beds, and one or more reaction zones may be within the same plant. Different plants are typically geographically separated so that feedstocks cannot be transferred between them without an intervening transportation and storage step. Accordingly, in various embodiments as otherwise described herein, the first reaction zone comprises a first reactor in which the hydrogenation catalyst is disposed, the second reaction zone comprises a second reactor in which the methanol decomposition catalyst is disposed, and wherein the third reaction zone comprises a third reactor in which the Fischer-Tropsch catalyst is disposed. In various embodiments as otherwise described herein, the first reaction zone comprises a first catalyst bed in which the hydrogenation catalyst is disposed, the second reaction zone comprises a second catalyst bed in which the methanol decomposition catalyst is disposed, and the third reaction zone comprises a third catalyst bed in which the Fischer-Tropsch catalyst is disposed. For example, in particular embodiments, the first catalyst bed, the second catalyst bed and the third catalyst bed are disposed within the same reactor, e.g., in catalyst stacks or catalyst bed sections. In some embodiments, the second reactor bed and the third reactor bed are disposed within the same reactor and the first catalyst bed is disposed in a different reactor than the second and third catalyst beds. For example, in particular embodiments, the first reactor is located in a first plant, and the second and third reactors are located in a second plant.

In certain embodiments, similar catalysts may be employed for some reactions. For example, in some embodiments, the methanol decomposition catalyst and the Fischer-Tropsch synthesis catalyst have the same composition. In particular embodiments, the methanol decomposition catalyst and the Fischer-Tropsch synthesis catalyst are the same catalyst.

An example embodiment is shown in schematic view in FIG. 1, which depicts a process 100 that is contained within a single plant 141. First feed stream 111 comprising H₂ and CO₂ is conducted to hydrogenation reaction zone 110 (here, a hydrogenation reactor), and is contacted therein with a hydrogenation catalyst 113, under conditions appropriate to generate first product stream 112 comprising methanol. The first product stream will typically contain other substances as well. For example, in the embodiment of FIG. 1, the first product stream includes CO₂; at least a portion of the CO₂ is separated from the first product stream and recycled to the first feed stream 111 via CO₂ recycle 114. Similarly, the first product stream can include H₂ (e.g., unreacted H₂) and/or CO (e.g., from a recycle stream from the third product stream). Here, at least a portion of H₂ and/or CO is separated from the first product stream and recycled to the first feed stream 111 via H₂ and/or CO recycle stream 115. In the embodiment of FIG. 1, H₂ bypass 124 removes at least a portion of H₂ from the first product stream 112 and introduces it into the third feed stream 131 for use as a Fischer-Tropsch reactant. H₂ activation stream 134 removes at least a portion of H₂ from the first product stream 112 and introduces it into the third reaction zone for activation of the Fischer-Tropsch catalyst 133 as needed. The embodiment of FIG. 1 exhibits three different uses for hydrogen separated from the first product stream 112; the person of ordinary skill in the art will appreciate that many real-world processes would include none, one, two or all three of these. Any water present can be removed from the first product stream 112 by water separation stream 116, to give a predominantly dry second feed stream (121) and optionally the water feed stream 116 can be fed to an electrolysis reactor 160.

In the embodiment of FIG. 1, the remaining portions of the first product stream 112 (i.e., after the separations described above) are provided to the second feed stream 121. Second feed stream 121 includes at least a portion of the methanol of the first product stream, and is conducted to the methanol decomposition reaction zone 120 (here, a methanol decomposition reactor), in which it is contacted with methanol decomposition catalyst 123 to decompose at least a portion of the methanol to form a second product stream 122 comprising CO and H₂. The second feed stream 121 may also be augmented through the introduction of other gases, such as CO and/or H₂ through an augmenting feed (not shown). In the embodiment of FIG. 1, from the second reaction zone 120, second product stream 122 is conducted to third feed stream 131. Third feed stream 131 comprises H₂ (e.g., from second product stream) and at least a portion of the CO of the second product stream. Third feed stream 131 is conducted to a Fischer-Tropsch reaction zone 130 (here, a Fischer-Tropsch reactor) in which Fischer-Tropsch catalyst 133 is disposed. The third feed stream 131 may also be augmented through the introduction of other gases, such as CO and/or H₂ through an augmenting feed (not shown). The third feed stream 131 is contacted with the Fischer-Tropsch catalyst 133 to provide a third product stream 132 that comprises C₅₊ hydrocarbons. In the embodiment of FIG. 1, third product stream 132 is conducted from the third reaction zone 130. Here, a light hydrocarbon stream 136 separated from the third product stream 132 and conducted to electrical generator 170. Additionally or alternatively, a light hydrocarbon stream 137, optionally comprising gas phase products, is separated from the third product stream 132 and recycled back to third feed stream 131. Examples of gas phase products for recycling include CO, H₂, CH₄, CO₂, C₂H₄, C₃H₆, C₃H₈, C₃H₇OH and H₂O. CO₂ recycle stream 138 may be used to recycle CO₂ from the third product stream 132 to the first feed stream 111. The electrical generator 170 uses the light hydrocarbon stream to create electrical power 172 (e.g., through burning the light hydrocarbon stream to form steam, which in turn can drive a turbine), which is provided to electrolysis reactor 160. As the person of ordinary skill in the art will appreciate, the Fischer-Tropsch reaction produces water as a byproduct; as shown in FIG. 1, at least a portion of the water can be separated from the third product stream 132, which can be conducted via water stream 138 to electrolysis reactor 160. The electrolysis reactor electrolyzes water (here, separated from the first product stream and/or the third product stream) to provide hydrogen gas using electrical power derived at least in part from the light hydrocarbon stream (although other sources of electrical power, including renewable sources, can be used). H₂ gas from the electrolysis reactor 160 is returned to the first feed stream by electrolysis H₂ stream 117.

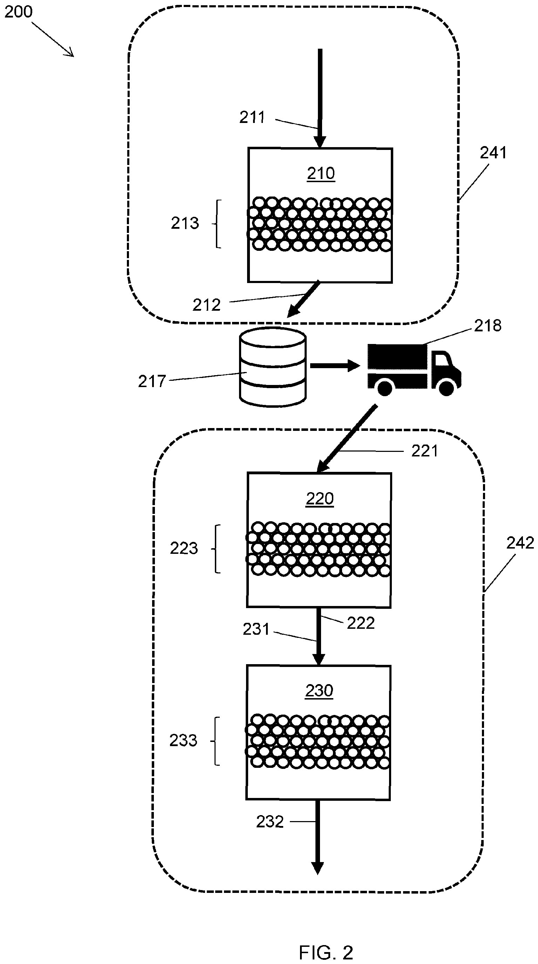

As noted above, methanol can be formed via hydrogenation of carbon dioxide in a first plant, then transported (e.g., via vehicle or pipeline) to a second plant where the methanol is decomposed and the resulting carbon monoxide is used in a Fischer-Tropsch reaction to provide hydrocarbons. An example of such an embodiment is shown in schematic view in FIG. 2. Here, the carbon dioxide hydrogenation step of process 200 is performed in a first plant 241. A first feed stream 211 comprising carbon dioxide and hydrogen is conducted to a hydrogenation reaction zone 210 (here, a hydrogenation reactor) containing CO₂ hydrogenation catalyst 213. The first feed stream 211 is contacted with the CO₂ hydrogenation catalyst 213 to hydrogenate at least a portion of the CO₂ to generate a first product stream 212 that includes methanol. First product steam 212 can be subjected to various separations as described herein, not shown, and then methanol of the first product stream can be stored for later use, and/or or transported to another plant. For example, in the embodiment of FIG. 2, at least a portion of the first product stream 212, the portion comprising methanol, is loaded into storage tank 217, then transported to second plant 243, here, by truck 218. The portion of the first product stream is provided to second feed stream 221, which is conducted to methanol decomposition reaction zone 220, in which it is contacted with methanol decomposition catalyst 223 to generate second product stream 222 comprising CO and H₂. Here, at least a portion of second product stream 222 is provided to third feed stream 231. Third feed stream 231 is conducted to Fischer-Tropsch reaction zone 230, in which it is contacted with Fischer-Tropsch catalyst 233, resulting in third product stream 232 comprising C₅₊ hydrocarbons.

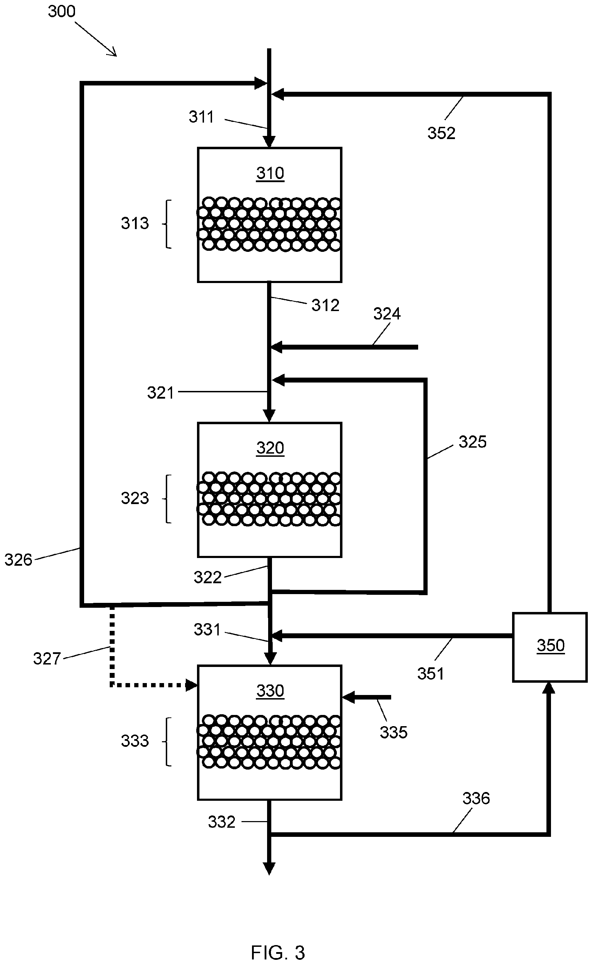

Another example embodiment is shown in schematic view in FIG. 3. Here, in process 300 first feed stream 311 is conducted to first reaction zone 310 and contacted with CO₂ hydrogenation catalyst 313 to hydrogenate at least a portion of CO₂ of the first feed stream to form first product stream 312 comprising methanol. First product stream 312 is conducted from the first reaction zone 310 and additional gas stream 324 is added, and the resulting mixture is provided as second feed stream 321. Second feed stream 321 conducted to methanol decomposition reaction zone 320 in which with methanol decomposition catalyst 323 to provide second product stream 322. Here, second product stream 322 is conducted from the methanol decomposition reaction zone, and unreacted methanol is substantially separated from the second product stream (e.g., at least 50%, at least 75%, at least 90% or at least 95%) and is recycled to the second feed stream 321 via methanol recycle 325. Notably, at least a portion of H₂ of the second product stream 322 can be separated and recycled to the first feed stream 311 via H₂ recycle 326. At least a portion of H₂ of the second product stream 322 may also or alternatively be provided to the third reaction zone 330 to activate Fischer-Tropsch catalyst 333 through H₂ stream 327. The rest of second product stream 322 is provided as part of third feed stream 331, which is conducted into Fischer-Tropsch reaction zone 330. Also part of third feed stream 331 is additional gas stream 335, which provides additional H₂ and/or CO to the Fischer-Tropsch reaction zone through a separate inlet. The third feed stream is contacted with Fischer-Tropsch catalyst 333 in the Fischer-Tropsch reaction zone to provide a third product stream 332 that includes C₅₊ hydrocarbons. Third product stream 332 is conducted from the Fischer-Tropsch reaction zone, and a light hydrocarbon stream 336 is separated therefrom and conducted to partial oxidation unit 350. The partial oxidation unit, as the person of ordinary skill in the art would appreciate, oxidizes the light hydrocarbons to provide CO2 and CO; at least a portion of CO generated from the partial oxidation unit 350 is included in the third feed stream 331 via CO stream 351. At least a portion of CO₂ generated from the partial oxidation unit 350 is included in the first feed stream 311 via CO₂ stream 352.

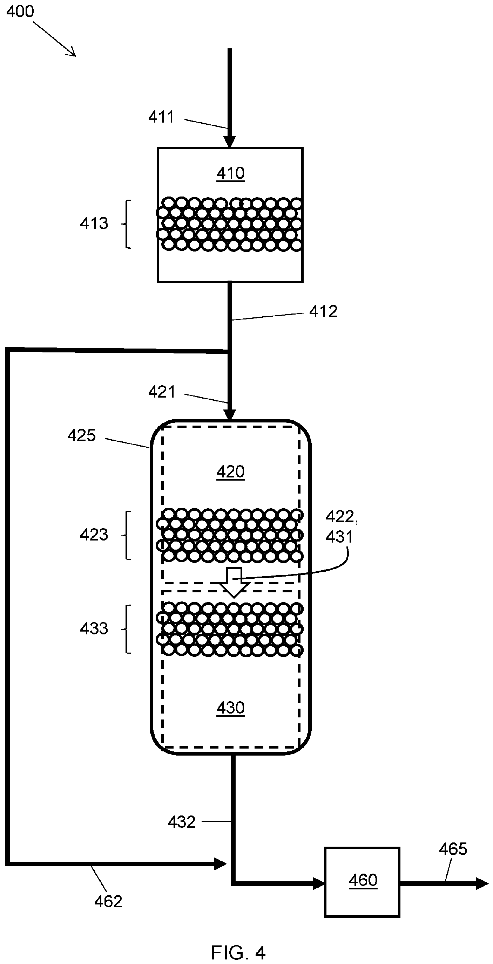

As noted above, various catalysts can be provided together in the same reactor. An example of one such embodiment is shown in schematic view in FIG. 4. Here, in process 400, the CO₂ hydrogenation is performed in one reactor and both the methanol decomposition and the Fischer-Tropsch synthesis are performed in another reaction. First feed stream 411 including CO₂ and H₂ is conducted to first reaction zone 410 (here, a hydrogenation reactor) and is contacted with hydrogenation catalyst 413 to provide a first product stream comprising methanol. First product stream 412 is provided as second feed stream 421 to stacked bed reactor 425, which includes a methanol decomposition reaction zone 420 (here, a first bed of the stacked-bed reactor that includes the methanol decomposition catalyst 423) and a Fischer-Tropsch reaction zone 430 (here, a second bed of the stacked-bed reactor that includes the Fischer-Tropsch catalyst 433). The second feed stream 421 is contacted with the methanol decomposition catalyst 423 to provide a second product stream 422 that includes CO and H₂. The second product stream is provided to the Fischer-Tropsch reaction zone as third feed stream 431, which is contacted with Fischer-Tropsch catalyst 433 to provide a third product stream 432 that includes C₅₊ hydrocarbons. Here, at least a portion of the C₅₊ hydrocarbons of the third product stream is conducted to hydroprocessing unit 460, where it is hydroprocessed to provide an end product stream 465. Here, at least a portion of H₂ of the first product stream is separated and conducted to the hydrotreating unit 460 via stream 462. H₂ can also be provided to the hydrotreating unit in the portion of the third product stream that is conducted to the hydrotreating unit.

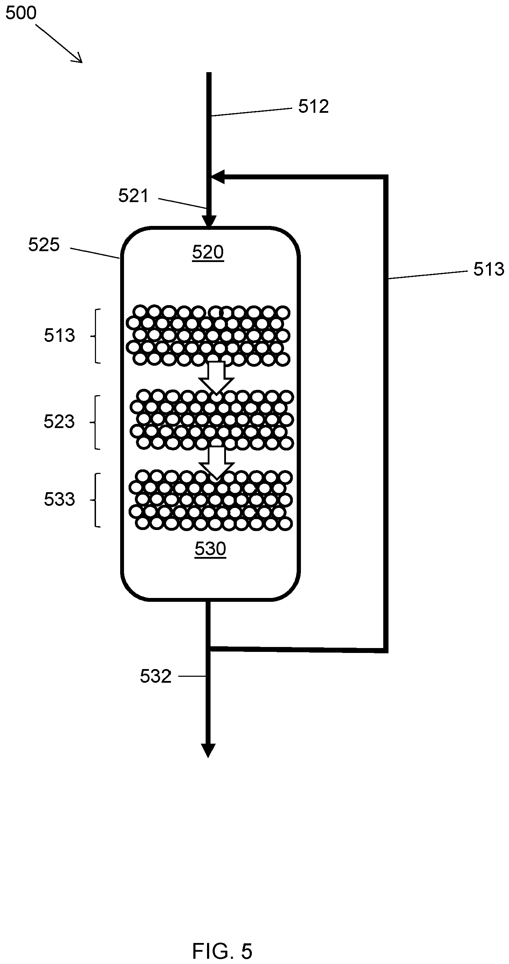

In some embodiments, the conversion of CO₂ to hydrocarbons through a methanol intermediate may be performed in a single reactor with various catalysts, for example, arranged in series. In the embodiment of FIG. 5, process 500 utilizes syngas source stream 512 which enters reactor 525 as first feed stream 521 at zone 520. The first feed stream 521 is contacted with hydrogenation catalyst 513, and then directly transmitted to methanol decomposition catalyst 523, and finally to Fischer-Tropsch catalyst 533. The resulting gas mixture is withdrawn from zone 530 as third product stream 532. Recycle stream 513 withdraws CO₂ gas, in optional admixture with light hydrocarbons and other gases, and recycles to CO₂ source stream 512.

Various exemplary embodiments of the disclosure include, but are not limited to the enumerated embodiments listed below, which can be combined in any number and in any combination that is not technically or logically inconsistent.

Embodiment 1. A process for performing an integrated Fischer-Tropsch synthesis, the process comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion of the methanol to form a second product stream comprising CO and H₂;
providing a third feed stream comprising H₂ and at least a portion of the CO of the second product stream;
contacting the third feed stream with a Fischer-Tropsch catalyst (e.g., in a Fischer-Tropsch reaction zone) to perform a Fischer-Tropsch synthesis to provide a third product stream comprising C₅₊ hydrocarbons.

Embodiment 2. The process according to embodiment 1, wherein the first feed stream includes at least 10 mol% H₂, e.g., at least 20 mol% H₂, or at least 30 mol% H₂.

Embodiment 3. The process according to embodiment 1 or embodiment 2, wherein the first feed stream includes at least 5 mol% CO₂, e.g., at least 10 mol% CO₂, or at least 15 mol% CO₂.

Embodiment 4. The process according to any of embodiments 1-3, wherein a ratio of H₂:CO₂ of the first feed stream is at least 1:1, e.g.at least 1.5:1, at least 2:1, at least 2.5:1, at least 3:1, or at least 4:1.

Embodiment 5. The process according to any of embodiments 1-4, wherein the first feed stream further comprises one or more of CO, CH₄ and N₂.

Embodiment 6. The process according to any of embodiments 1-5, wherein the hydrogenation catalyst is a copper/zinc oxide catalyst, e.g., supported on aluminum oxide or zirconium oxide.

Embodiment 7. The process according to any of embodiments 1-6, wherein the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

Embodiment 8. The process according to any of embodiments 1-7, wherein the hydrogenation of CO₂ is performed with a selectivity of at least 50% for methanol, e.g., at least 65% or at least 80%.

Embodiment 9. The process according to any of embodiments 1-8, wherein the hydrogenation of CO₂ is performed with a selectivity of no more than 20% for methane, e.g., no more than 10%, or no more than 5%.

Embodiment 10. The process according to any of embodiments 1-9, wherein the hydrogenation of CO₂ is performed with a selectivity of no more than 20% for CO, e.g., no more than 10%, or no more than 5%.

Embodiment 11. The process according to any of embodiments 1-10, wherein the hydrogenation of CO₂ is performed with a conversion of CO₂ of at least 25%, e.g. at least 35%, e.g., at least 45% or at least 50%.

Embodiment 12. The process according to any of embodiments 1-11, wherein the first product stream includes at least 15 mol% methanol, e.g., at least 25 mol%, or at least 35 mol% methanol.

Embodiment 13. The process according to any of embodiments 1-12, wherein the first product stream includes no more than 10 mol% methane, e.g., no more than 5 mol% methane, or no more than 2 mol% methane.

Embodiment 14. The process according to any of embodiments 1-13, wherein the first product stream includes no more than 10 mol% CO, e.g., no more than 5 mol% CO, or no more than 2 mol% CO.

Embodiment 15. The process according to any of embodiments 1-14, further comprising separating at least a portion of water from the first product stream (e.g., at least 50%, at least 75%, or at least 90% of water in the first product stream).

Embodiment 16. The process of any of embodiments 1-15, wherein the portion of the first product stream that is included in the second feed stream has a water content of no more than 10 mol%, e.g., or no more than 2 mol%, or no more than 0.5 mol%.

Embodiment 17. The process according to any of embodiments 1-15, further comprising separating at least a portion of H₂ from the first product stream (e.g., at least 50%, or at least 60%, or at least 75%, or at least 90%, or at least 95% of H₂ in the first product stream).

Embodiment 18. The process of embodiment 17, further comprising recycling to the first feed stream at least a portion of the H₂ separated from the first product stream.

Embodiment 19. The process of embodiment 17 or embodiment 18, further comprising providing at least a portion of the H₂ separated from the first product stream to the third feed stream.

Embodiment 20. The process of any of embodiments 17-19, further comprising activating the Fischer Tropsch catalyst using at least a portion of the H₂ separated from the first product stream.

Embodiment 21. The process of any of embodiments 17-20, further comprising activating the methanol decomposition catalyst using at least a portion of the H₂ separated from the first product stream.

Embodiment 22. The process of any of embodiments 1-21, wherein the portion of the first product stream that is provided to the second feed stream includes no more than 50% of the hydrogen of the first product stream, e.g., no more than 25%, no more than 10%, or no more than 5% of the hydrogen of the first product stream.

Embodiment 23. The process of any of embodiments 1-22, wherein the contacting of the second feed stream with the methanol decomposition catalyst and the contacting of the first feed stream with the hydrogenation catalyst are performed in the same plant.

Embodiment 24. The process according to any of embodiments 1-23, wherein at least a portion of methanol of the first product stream is provided directly to the second feed stream.

Embodiment 25. The process according to embodiment 24, wherein the portion of the first product stream that is provided to the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 C, or 300-400 °C.

Embodiment 26. The process according to any of embodiments 1-23, wherein the contacting of the second feed stream with the methanol decomposition catalyst and the contacting of the first feed stream with the hydrogenation catalyst are performed in different plants.

Embodiment 27. The process according to any of embodiments 1-26, wherein at least a portion of methanol of the first product stream is stored (e.g., in one or more tanks) before provision to the second feed stream.

Embodiment 28. The process according to embodiment 27, wherein the stored portion of the methanol of the first product stream is stored for at least one day, e.g., at least two days or at least a week.

Embodiment 29. The process according to embodiment 27 or embodiment 28, wherein the stored portion of the methanol is transported at least 1 km during storage, e.g., at least 2 km or at least 10 km.

Embodiment 30. The process according to any of embodiments 1-29, wherein the second feed stream comprises at least 5 mol% methanol, e.g., at least 7.5% methanol, or at least 10 mol% methanol, or at least 15% methanol, or at least 20% methanol, or at least 25 mol% methanol.

Embodiment 31. The process according to any of embodiments 1-30, wherein the second feed stream further comprises one or more of H₂, CO, CH₄, CO₂ and N₂.

Embodiment 32. The process according to any of embodiments 1-31, wherein the second feed stream has a concentration of water of no more than 5 mol%, e.g., no more than 2 mol% or no more than 1 mol%.

Embodiment 33. The process according to any of embodiments 1-32, wherein the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

Embodiment 34. The process according to any of embodiments 1-33, wherein the methanol decomposition catalyst is a copper/zinc oxide catalyst, e.g., on alumina.

Embodiment 35. The process according to any of embodiments 1-34, wherein the contacting of the second feed stream with the methanol decomposition catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

Embodiment 36. The process according to any of embodiments 1-35, wherein the contacting of the second feed stream with the methanol decomposition catalyst is performed at a temperature within 75 °C of a temperature of the contacting of the first feed stream with the hydrogenation catalyst, e.g., within 50 °C.

Embodiment 37. The process according to any of embodiments 1-36, wherein the decomposition of methanol is performed with a carbon product selectivity of at least 50% for CO, e.g., at least 60%, or at least 70%, or at least 80%, or at least 90%.

Embodiment 38. The process according to any of embodiments 1-37, wherein the decomposition of methanol is performed with a selectivity of no more than 20% for CO₂, e.g., no more than 15%, no more than 10%, or no more than 5%.

Embodiment 39. The process according to any of embodiments 1-38, wherein the decomposition of methanol is performed with a conversion of methanol of at least 30%, e.g. at least 40%, or at least 50%, or at least 60%, or at least 65%, or at least 70%, or at least 75%.

Embodiment 40. The process according to any of embodiments 1-39, wherein the second product stream includes at least 20 mol% total of CO and H₂, e.g., at least 35 mol%, or at least 50 mol%, or at least 65 mol%.

Embodiment 41. The process according to any of embodiments 1-40, wherein the second product stream has a molar ratio of hydrogen to carbon monoxide in the range of 0.5:1 to 5:1, e.g., 1:1 to 3:1, 1.5:1 to 2.5:1.

Embodiment 42. The process according to any of embodiments 1-41, wherein the second product stream includes no more than 75 mol% methanol, e.g., no more than 60 mol% methanol, or no more than 50 mol% methanol, or no more than 25 mol% methanol.

Embodiment 43. The process according to any of embodiments 1-42, further comprising separating at least a portion of methanol from the second product stream (e.g., at least 50%, at least 75%, or at least 90% of methanol in the second product stream).

Embodiment 44. The process of embodiment 43, further comprising recycling to the second feed stream at least a portion of the methanol separated from the second product stream, e.g., at least 50%, or at least 75%, or at least 90%, or at least 95% of the methanol from the second product stream.

Embodiment 45. The process of any of embodiments 1-44, further comprising separating at least a portion of H₂ from the second product stream.

Embodiment 46. The process of embodiment 45, further comprising providing the H₂ separated from the second product stream to the first feed stream.

Embodiment 47. The process of any of embodiments 45 and 46, further comprising activating the Fischer Tropsch catalyst using at least a portion of the H₂ separated from the second product stream.

Embodiment 48. The process of any of embodiments 1-47, wherein at least 50% of CO of the second product stream is provided to the third feed stream, e.g., at least 75%, or at least 90%%, or at least 95%, or at least 99%.

Embodiment 49. The process of any of embodiments 1-48, wherein at least a portion of H₂ of the second product stream is provided to the third feed stream.

Embodiment 50. The process of any of embodiments 1-49, wherein at least 50% of H₂ of the second product stream is provided to the third feed stream, e.g., at least 75%, or at least 90%, or at least 95%, or at least 99%.

Embodiment 51. The process of any of embodiments 1-50, wherein H₂ is provided to the third feed stream from a hydrogen source other than the second product stream.

Embodiment 52. The process of any of embodiments 1-51, wherein CO is provided to the third feed stream from a CO source other than the second product stream.

Embodiment 53. The process of any of embodiments 1-52, wherein one or more of CO₂, CH₄ and N₂ is provided to the third feed stream from a source thereof other than the second product stream.

Embodiment 54. The process of any of embodiments 1-53, wherein the portion of the second product stream that is included in the third feed stream has a H₂:CO ratio in the range of 0.5:1 to 5:1, e.g., in the range of 1:1 to 2.5:1.

Embodiment 55. The process of any of embodiments 1-54, wherein the third feed stream has a molar H₂:CO ratio in the range of 0.5:1 to 5:1.

Embodiment 56. The process of any of embodiments 1-55, wherein the third feed stream has a molar H₂:CO ratio in the range of 1:1 to 2.5:1.

Embodiment 57. The process of any of embodiments 1-56, wherein the third feed stream has a molar H₂:CO ratio of at least 1.2:1, e.g., in the range of 1.2:1 - 2.5:1.

Embodiment 58. The process of any of embodiments 1-57, wherein the Fischer-Tropsch catalyst comprises cobalt, iron, rhodium, ruthenium, or a combination thereof.

Embodiment 59. The process of any of embodiments 1-58, wherein the Fischer-Tropsch catalyst comprises cobalt in an amount in the range of 5-25 wt%, calculated as Co(0).

Embodiment 60. The process of any of embodiments 1-59, wherein the Fischer-Tropsch catalyst comprises iron in an amount in the range of 25-95 wt%, calculated as Fe(0).

Embodiment 61. The process of any of embodiments 58-60, wherein the Fischer-Tropsch catalyst further comprises manganese.

Embodiment 62. The process of any of embodiments 1-61, wherein the Fischer-Tropsch catalyst is a supported catalyst, wherein the support comprises at least one of titanium oxide, zirconium oxide, cerium oxide, aluminum oxide, magnesium oxide, silicon oxide and zinc oxide.

Embodiment 63. The process of any of embodiments 1-62, wherein the Fischer-Tropsch catalyst is a supported catalyst, wherein the support comprises at least one of titanium oxide, aluminum oxide, and silicon oxide.

Embodiment 64. The process of any of embodiments 1-63, wherein the Fischer-Tropsch catalyst is a supported catalyst, wherein the support is a titanium dioxide support.

Embodiment 65. The process of any of embodiments 1-64, wherein the Fischer-Tropsch catalyst is activated in a reductive atmosphere.

Embodiment 66. The process of embodiment 65, wherein the reductive atmosphere comprises at least a portion of hydrogen from the second product stream and/or at least a portion of hydrogen of the first product stream.

Embodiment 67. The process of any of embodiments 1-66, wherein the contacting of the third feed stream with the Fischer-Tropsch catalyst is performed at a temperature in the range of 150-400 °C (e.g., in the range of 150-350 °C, or 150-300 °C, or 150-250 °C, or 150-200 °C, or 200-400 °C, or 200-350 °C, or 200-300 °C, or 200-250 °C, or 250-400 °C, or 250-350 °C, or 250-300 °C, or 300-400 °C).

Embodiment 68. The process of any of embodiments 1-67, wherein the contacting of the third feed stream with the Fischer-Tropsch catalyst is performed at a temperature in the range of 200-350 °C.

Embodiment 69. The process of any of embodiments 1-68, wherein the contacting of the third feed stream with the Fischer-Tropsch catalyst is performed at a pressure in the range of 10-50 barg (e.g., 20-50 barg, or 25-50 barg, or 10-40 barg, or 20-40 barg, or 25-40 barg or 10-35 barg, or 20-35 barg, or 25-35 barg).

Embodiment 70. The process of any of embodiments 1-69, wherein the contacting of the third feed stream with the Fischer-Tropsch catalyst is performed at a pressure in the range of 20-40 barg.

Embodiment 71. The process of any of embodiments 1-70, wherein the contacting of third feed stream with the Fischer-Tropsch catalyst and the contacting of the second feed stream with the methanol decomposition catalyst are performed in the same plant.

Embodiment 72. The process of any of embodiments 1-71, wherein the contacting of the Fischer-Tropsch catalyst with the third feed stream is performed with a C₅₊ selectivity of at least 30%, e.g., at least 50%, or at least 70%.

Embodiment 73. The process of any of embodiments 1-72, wherein the C₅₊ hydrocarbons of the third product stream comprise C₅₊ oxygenates.

Embodiment 74. The process of any of embodiments 1-73, further comprising separating at least a portion of C₁-C₄ hydrocarbons from the third product stream to provide a light hydrocarbon stream.

Embodiment 75. The process of embodiment 74, further comprising oxidizing at least a portion of the light hydrocarbon stream in a partial oxidation reactor to provide a pOX stream comprising CO and/or CO₂, and including at least a portion of the pOX stream in the first feed stream or the third feed stream.

Embodiment 76. The process of embodiment 74 or embodiment 75, further comprising including at least a portion of the light hydrocarbon stream in the third feed stream.

Embodiment 77. The process of any of embodiments 1-76, further comprising separating at least a portion of H₂ of the third product stream.

Embodiment 78. The process of embodiment 77, wherein at least a portion of the H₂ separated from the third product stream is provided to the first feed stream and/or to the third feed stream.

Embodiment 79. The process of embodiment 77 or embodiment 78, wherein at least a portion of the H₂ separated from the third product stream is used to activate the Fischer-Tropsch catalyst.

Embodiment 80. The process of any of embodiments 1-79, further comprising separating at least a portion of CO of the third product stream.

Embodiment 81. The process of embodiment 80, wherein at least a portion of the CO separated from the third product stream is provided to the third feed stream.

Embodiment 82. The process of any of embodiments 1-81, further comprising separating at least a portion of CO₂ of the third product stream

Embodiment 83. The process of embodiment 82, wherein at least a portion of the CO₂ separated from the third product stream is provided to the first feed stream.

Embodiment 84. The process of any of embodiments 1-83, further comprising providing from the third product stream a C₅₊ hydrocarbon product stream comprising at least 80 wt% C₅₊ hydrocarbons, e.g., at least 90 wt% C₅₊ hydrocarbons.

Embodiment 85. The process of any of embodiments 1-84, further comprising hydroprocessing at least a portion of the C₅₊ hydrocarbon third product stream by contacting the C₅₊ hydrocarbon product stream with hydrogen and a hydroprocessing catalyst.

Embodiment 86. The process of embodiment 85, wherein at least a portion of the hydrogen used in the hydroprocessing is provided from any of the first product stream, the second product stream and the third product stream.

Embodiment 87. The process of any of embodiments 1-86, wherein one or more products are provided from at least a portion of C₅₊ hydrocarbons of the third product stream, the one or more products being one or more of a fuel, a wax, or an oil.

Embodiment 88. The process of any of embodiments 1-87, wherein at least part of the CO₂ of the first feed stream is from a renewable source.

Embodiment 89. The process of any of embodiments 1-88, wherein at least part of the CO₂ of the first feed stream is from direct air capture.

Embodiment 90. The process of any of embodiments 1-89, wherein at least part of the CO₂ of the first feed stream is captured from a manufacturing plant, e.g., a bioethanol plant, a steel plant or a cement plant.

Embodiment 91. The process of any of embodiments 1-90, wherein at least part of the H₂ of the first feed stream and/or the third feed stream is from a renewable source.

Embodiment 92. The process of any of embodiment 1-91, wherein at least a portion of the hydrogen of the first feed stream and/or the third feed stream is green hydrogen.

Embodiment 93. The process of any of embodiment 1-92, wherein at least a portion of the hydrogen of the first feed stream and/or the third feed stream is blue hydrogen.

Embodiment 94. The process of any of embodiment 1-93, wherein at least a portion of the hydrogen of the first feed stream and/or the third feed stream is grey hydrogen, black hydrogen, brown hydrogen, pink hydrogen, turquoise hydrogen, yellow hydrogen, and/or white hydrogen.

Embodiment 95. The process of any of embodiments 1-94, further comprising providing at least a portion of H₂ to the first feed stream and/or the third feed stream by electrolysis of water.

Embodiment 96. The process of embodiment 95, wherein the electrolysis of water is performed using electricity at least partially derived from a renewable source.

Embodiment 97. The process of embodiment 95 or embodiment 96, wherein the electrolysis of water is performed using electricity at least partially generated from steam made by combustion of a light hydrocarbon stream provided from the third product stream.

Embodiment 98. The process of any of embodiments 95-97, further comprising the step of embodiment 75, wherein at least a portion of O₂ generated by the water electrolysis is provided to the partial oxidation reactor.

Embodiment 99. The process of any of embodiments 1-98, wherein the first reaction zone comprises a first reactor in which the hydrogenation catalyst is disposed, the second reaction zone comprises a second reactor in which the methanol decomposition catalyst is disposed, and wherein the third reaction zone comprises a third reactor in which the Fischer-Tropsch catalyst is disposed.

Embodiment 100. The process of any of embodiments 1-99, wherein the first reaction zone comprises a first catalyst bed in which the hydrogenation catalyst is disposed, the second reaction zone comprises a second catalyst bed in which the methanol decomposition catalyst is disposed, and the third reaction zone comprises a third catalyst bed in which the Fischer-Tropsch catalyst is disposed.

Embodiment 101. The process of embodiment 100, wherein the first catalyst bed, the second catalyst bed and the third catalyst bed are disposed within the same reactor in catalyst stacks or bed sections.

Embodiment 102. The process of embodiment 101, wherein the second catalyst bed and the third catalyst bed are disposed within the same reactor and the first catalyst bed is disposed in a different reactor than the second and third catalyst beds.

Embodiment 103. The process of any of embodiments 1-102, wherein the methanol decomposition catalyst and the Fischer-Tropsch synthesis catalyst have the same composition (e.g., are the same catalyst) .

The particulars shown herein are by way of example and for purposes of illustrative discussion of various embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains various errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for performing an integrated Fischer-Tropsch synthesis, the process comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion of the methanol to form a second product stream comprising CO and H₂;
providing a third feed stream comprising H₂ and at least a portion of the CO of the second product stream;
contacting the third feed stream with a Fischer-Tropsch catalyst (e.g., in a Fischer-Tropsch reaction zone) to perform a Fischer-Tropsch synthesis to provide a third product stream comprising C₅₊ hydrocarbons.

2. The process according to claim 1, wherein the first product stream includes at least 15 mol% methanol, e.g., at least 25 mol%, or at least 35 mol% methanol.

3. The process according claim 1 or claim 2, further comprising separating at least a portion of water from the first product stream (e.g., at least 50%, at least 75%, or at least 90% of water in the first product stream).

4. The process according to any of claims 1-3, wherein the portion of the first product stream that is included in the second feed stream has a water content of no more than 10 mol%, e.g., or no more than 2 mol%, or no more than 0.5 mol%.

5. The process according to any of claims 1-4, wherein the second feed stream comprises at least 5 mol% methanol, e.g., at least 7.5% methanol, or at least 10 mol% methanol, or at least 15% methanol, or at least 20% methanol, or at least 25 mol% methanol,

6. The process according to any of claims 1-5, wherein the second feed stream has a concentration of water of no more than 5 mol%, e.g., no more than 2 mol% or no more than 1 mol%.

7. The process according to any of claims 1-6, wherein the contacting of the second feed stream with the methanol decomposition catalyst is performed at a temperature within 75 °C of a temperature of the contacting of the first feed stream with the hydrogenation catalyst, e.g., within 50 °C.

8. The process according to any of claims 1-7, further comprising separating at least a portion of methanol from the second product stream (e.g., at least 50%, at least 75%, or at least 90% of methanol in the second product stream), and recycling to the second feed stream at least a portion of the methanol separated from the second product stream, e.g., at least 50%, or at least 75%, or at least 90%, or at least 95% of the methanol from the second product stream.

9. The process of any of claims 1-8, further comprising separating at least a portion of H₂ from the second product stream, and providing the H₂ separated from the second product stream to the first feed stream.

10. The process of any of claims 1-9, wherein the portion of the second product stream that is included in the third feed stream has a H₂:CO ratio in the range of 0.5:1 to 5:1, e.g., in the range of 1:1 to 2.5:1, e.g., in the range of 1.5:1 to 3.5:1.

11. The process of any of claims 1-10, wherein the contacting of third feed stream with the Fischer-Tropsch catalyst and the contacting of the second feed stream with the methanol decomposition catalyst are performed in the same plant.

12. The process of any of claims 1-11, further comprising separating at least a portion of C₁-C₄ hydrocarbons from the third product stream to provide a light hydrocarbon stream, and oxidizing at least a portion of the light hydrocarbon stream in a partial oxidation reactor to provide a pOX stream comprising CO and/or CO₂, and including at least a portion of the pOX stream in the first feed stream or the third feed stream.

13. The process according to claim 12, further comprising including at least a portion of the light hydrocarbon stream in the third feed stream.

14. The process of any of claims 1-13, further comprising separating at least a portion of H₂ of the third product stream, wherein at least a portion of the H₂ separated from the third product stream is provided to the first feed stream and/or to the third feed stream.

15. The process of any of claims 1-14, further comprising separating at least a portion of CO₂ of the third product stream, wherein at least a portion of the CO₂ separated from the third product stream is provided to the first feed stream.
